# EUROPEAN PATENT APPLICATION

(11) **EP 4 524 132 A1**
(43) Date of publication of application: **19.03.2025**
(21) Application number: 23803581.0
(22) Date of filing: 10.05.2023
(51) Int. Cl.: C07D 277/10, A61K 31/426, A61P 9/10, A61P 29/00

(54) **2-METHYL-2-THIAZOLINE SALT**

(30) Priority: 10.05.2022 JP 2022077593
(71) Applicant: Myrodia Therapeutics Co., Ltd., Tokyo 107-0061 (JP)
(72) Inventor: TANIGAWA, Kiyoshi, Tokyo 107-0061 (JP); MARUHASHI, Kazuo, Tokyo 107-0061 (JP)
(74) Representative: Weickmann & Weickmann PartmbB
(86) International application number: PCT/JP2023/017574
(87) International publication number: WO 2023/219106

(57) **Abstract**

A solid derivative of 2-methyl-2-thiazoline which is easy to handle during formulation and is useful as an active pharmaceutical ingredient for pharmaceutical products is provided. A 1,5-naphthalenedisulfonic acid salt or a saccharin salt of 2-methyl-2-thiazoline, or a deuterated form thereof, a pharmaceutical composition containing the same, a prophylactic or therapeutic agent for hypoxic injury, ischemia-reperfusion injury or inflammation, which contains the same, and a method for producing a 1,5-naphthalenedisulfonic acid salt or a saccharin salt of 2-methyl-2-thiazoline, or a deuterated form thereof.

## Description

### [Technical Field]

The present invention relates to a salt of 2-methyl-2-thiazoline which is useful as a medicament, for example, a prophylactic or therapeutic agent for hypoxic injury, ischemia-reperfusion injury or inflammation, and a production method thereof.

### [Background Art]

### 2-Methyl-2-thiazoline represented by the formula (I)

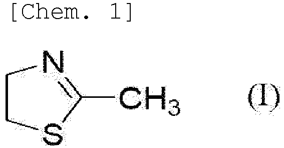

(hereinafter sometimes to be referred to as "2-MT") is a compound useful as a medicament, for example, a prophylactic or therapeutic agent for hypoxic injury, ischemia-reperfusion injury or inflammation (Patent Literature 1). However, 2-MT is a highly volatile liquid with a pungent odor, which makes it difficult to handle during formulation. In addition, when weighing, it is difficult to accurately measure the weight of 2-MT.

### [Citation List]

### [Patent Literature]

[Patent Literature 1]
WO 2019/177142

### [Summary of Invention]

### [Technical Problem]

The present invention aims to provide a solid derivative of 2-MT that is easy to handle during formulation and is useful as an active pharmaceutical ingredient of pharmaceutical products.

### [Solution to Problem]

The present inventors have conducted intensive studies in an attempt to solve the above-mentioned problems and found that a salt of 2-MT with 1,5-naphthalenedisulfonic acid (hereinafter sometimes to be referred to as "NDSA") or saccharin can be obtained as a solid excellent in handling property and stability as an active pharmaceutical ingredient of pharmaceutical products, which resulted in the completion of the present invention.

It is known that deuterated compounds, in which the C-H bond at the metabolic site of a medicament molecule is replaced with a more stable C-D bond, can slow down metabolism and are less likely decomposed due to the deuterium isotope effect; on the other hand, the properties of the entire molecule are hardly influenced and the biological activity can be retained. According to the present invention, a deuterated form of the NDSA salt or saccharin salt of 2-MT is provided.

That is, the present invention relates to the following.
[1] A 1,5-naphthalenedisulfonic acid salt or a saccharin salt of 2-methyl-2-thiazoline, or a deuterated form thereof.
[2] The salt of [1], wherein the salt is a 1,5-naphthalenedisulfonic acid salt or a saccharin salt of 2-methyl-2-thiazoline.
[3] The salt of [1] or [2], wherein the salt is a crystal.
[4] The salt of [1], wherein the salt is a 1,5-naphthalenedisulfonic acid salt of 2-methyl-2-thiazoline, or a deuterated form thereof.
[5] The salt of [4], wherein the salt is a hemi-1,5-naphthalenedisulfonic acid salt of 2-methyl-2-thiazoline, or a deuterated form thereof.
[6] The salt of [5], wherein the salt is a hydrate of a hemi-1,5-naphthalenedisulfonic acid salt of 2-methyl-2-thiazoline.
[7] The salt of [5] or [6], wherein the salt is a crystal.
[8] The salt of [7], wherein the salt has a powder X-ray diffraction pattern with peaks at 2θ values of 17.2±0.2°, 17.9±0.2°, 18.7±0.2°, and 22.6±0.2°.
[9] The salt of [7], wherein the salt has a powder X-ray diffraction pattern with peaks at 2θ values of 11.6±0.2°, 17.2±0.2°, 17.9±0.2°, 18.7±0.2°, 20.1±0.2°, 22.6±0.2°, and 23.9±0.2°.
[9A] The salt of any one of [5] to [9], wherein the salt has an endothermic peak with an extrapolated onset temperature (onset temperature) of 218°C±3°C, preferably 218°C±1°C, in differential scanning calorimetry (DSC).
[9B] The salt of [9A], further having an endothermic peak with an extrapolated onset temperature (onset temperature) of 108°C±3°C, preferably 108°C±1°C, in differential scanning calorimetry (DSC).
[10] The salt of [4], wherein the salt is a mono-1,5-naphthalenedisulfonic acid salt of 2-methyl-2-thiazoline, or a deuterated form thereof.
[11] The salt of [10], wherein the salt is a hydrate of a mono-1,5-naphthalenedisulfonic acid salt of 2-methyl-2-thiazoline.
[12] The salt of [10] or [11], wherein the salt is a crystal.
[13] The salt of [12], wherein the salt has a powder X-ray diffraction pattern with peaks at 2θ values of 18.5±0.2°, 22.6±0.2°, 22.9±0.2°, and 23.4±0.2°.
[14] The salt of [12], wherein the salt has a powder X-ray diffraction pattern with peaks at 2θ values of 11.9±0.2°, 14.4±0.2°, 18.5±0.2°, 22.6±0.2°, 22.9±0.2°, and 23.4±0.2°.
[14A] The salt of any one of [10] to [14], wherein the salt has an endothermic peak with an extrapolated onset temperature (onset temperature) of 268°C±3°C, preferably 268°C±1°C, in thermogravimetric/differential thermal analysis (TG/DTA).
[14B] The salt of [14A], further having endothermic peaks with extrapolated onset temperatures (onset temperatures) of 115°C±3°C and 142°C±3°C, preferably 115°C±1°C and 142°C±1°C, in thermogravimetric/differential thermal analysis (TG/DTA).
[14C] The salt of any one of [4] to [14], [9A], [9B], [14A], and [14B], wherein the 1,5-naphthalenedisulfonic acid salt of 2-methyl-2-thiazoline has a purity of 95% or more, preferably 97% or more, more preferably 98% or more, further preferably 99% or more.
[15] The salt of [1], wherein the salt is a saccharin salt of 2-methyl-2-thiazoline, or a deuterated form thereof.
[16] The salt of [15], wherein the salt is a monosaccharin salt of 2-methyl-2-thiazoline, or a deuterated form thereof.
[17] The salt of [16], wherein the salt is an anhydrate of a monosaccharin salt of 2-methyl-2-thiazoline.
[18] The salt of [16] or [17], wherein the salt is a crystal.
[19] The salt of [18], wherein the salt has a powder X-ray diffraction pattern with peaks at 2θ values of 12.8±0.2°, 13.8±0.2°, 19.9±0.2°, and 26.2±0.2°.
[20] The salt of [18], wherein the salt has a powder X-ray diffraction pattern with peaks at 2θ values of 12.8±0.2°, 13.8±0.2°, 14.7±0.2°, 19.9±0.2°, 26.2±0.2°, and 29.3±0.2°.
[20A] The salt of any one of [15] to [20], wherein the salt has an endothermic peak with an extrapolated onset temperature (onset temperature) of 131°C±3°C, preferably 131°C±1°C, in thermogravimetric/differential thermal analysis (TG/DTA).
[20B] The salt of any one of [15] to [20] and [20A], wherein the saccharin salt of 2-methyl-2-thiazoline has a purity of 95% or more, preferably 97% or more, more preferably 98% or more, further preferably 99% or more.
[21] The deuterated form of [1], which is represented by the formula (1) 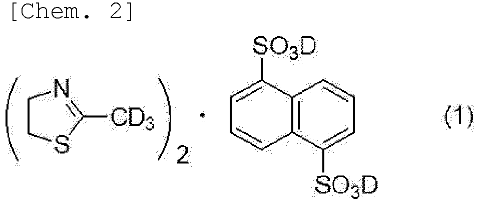 or the formula (2) 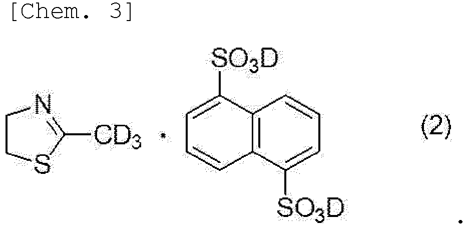
[22] The deuterated form of [1], which is represented by the formula (3) 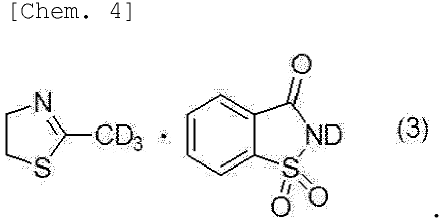 [23] A pharmaceutical composition comprising the salt or a deuterated form thereof of any one of [1] to [22], and a pharmaceutically acceptable carrier.
[24] A prophylactic or therapeutic agent for hypoxic injury, ischemia-reperfusion injury or inflammation, comprising the salt or a deuterated form thereof of any one of [1] to [22].
[24A] The salt or a deuterated form thereof of any one of [1] to [22] for use in the prophylaxis or treatment of hypoxic injury, ischemia-reperfusion injury or inflammation.
[24B] Use of the salt or a deuterated form thereof of any one of [1] to [22] for producing a prophylactic or therapeutic agent for hypoxic injury, ischemia-reperfusion injury or inflammation.
[24C] A method for preventing or treating hypoxic injury, ischemia-reperfusion injury or inflammation in a mammal, comprising administering an effective amount of the salt or a deuterated form thereof of any one of [1] to [22], to the mammal.
[25] A method for producing a 1,5-naphthalenedisulfonic acid salt or a saccharin salt of 2-methyl-2-thiazoline, comprising
   mixing 2-methyl-2-thiazoline, 1,5-naphthalenedisulfonic acid or saccharin, and a solvent, and
   isolating the obtained solid of the 1,5-naphthalenedisulfonic acid salt or saccharin salt of 2-methyl-2-thiazoline.
[26] A method for producing a deuterated form of a saccharin salt of 2-methyl-2-thiazoline, comprising
   mixing a saccharin salt of 2-methyl-2-thiazoline and deuterated methanol, and
   isolating the obtained deuterated form of the saccharin salt of 2-methyl-2-thiazoline.
[27] A method for producing a deuterated form represented by the formula (1) 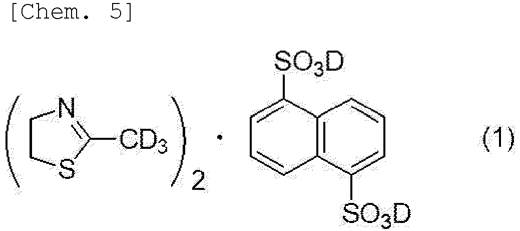 or the formula (2) 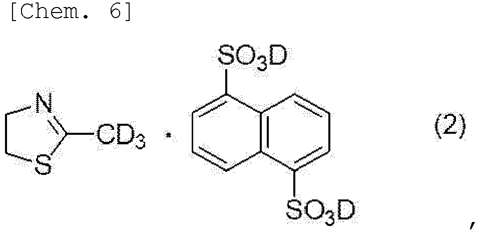 comprising mixing 2-methyl-d3-2-thiazoline and a deuterated form of 1,5-naphthalenedisulfonic acid in a solvent to obtain a deuterated form represented by the formula (1) or the formula (2).
[28] A method for producing a deuterated form represented by the formula (3) 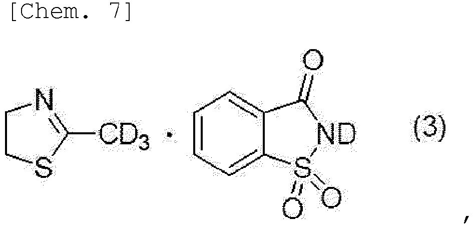 comprising mixing a saccharin salt of 2-methyl-2-thiazoline and deuterated methanol to obtain a deuterated form represented by the formula (3).

### [Advantageous Effects of Invention]

According to the present invention, a salt, particularly a crystalline salt, of 2-MT, which is a solid excellent in the handling property and stability as an active pharmaceutical ingredient of pharmaceutical products, is provided. The 2-MT salt of the present invention has high storage stability and is stable even when stored at high humidity. In addition, since the 2-MT salt of the present invention is a solid, the weight of the salt can be easily and accurately measured when weighing. The 2-MT salt of the present invention is useful as an active pharmaceutical ingredient of pharmaceutical products.

In the deuterated form of the present invention, the C-H bond is replaced with a more stable C-D bond, and therefore, the deuterium isotope effect can slow down metabolism and makes it difficult to decompose, while retaining biological activity. Therefore, when used as a medicament, it has the characteristics that the dosage and frequency of administration can be reduced, and side effects can be easily controlled. In addition, the deuterated form can be used as a tracer or internal standard substance in biological analyses (e.g., pharmacokinetic analysis and the like).

### [Brief Description of Drawings]

[Fig. 1]

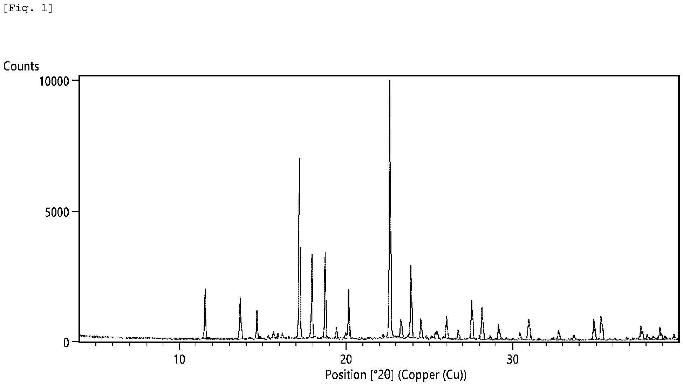

   Fig. 1 is a powder X-ray diffraction (XRPD) pattern of the hemi-NDSA salt of 2-MT produced in Example 1.
[Fig. 2]

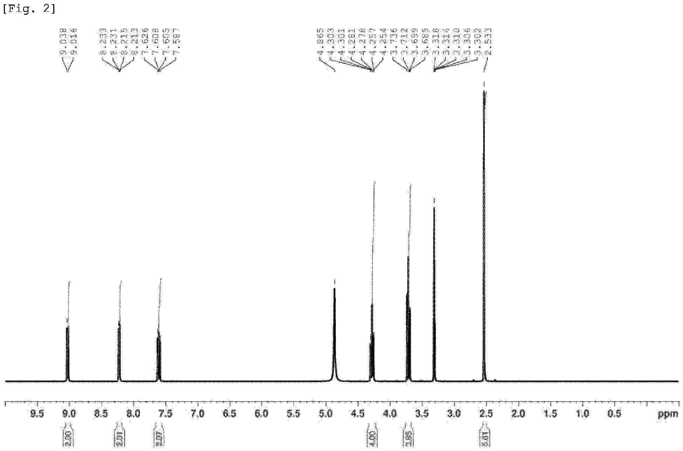

   Fig. 2 is a ¹H NMR spectrum (CD₃OD) of the hemi-NDSA salt of 2-MT produced in Example 1.
[Fig. 3]

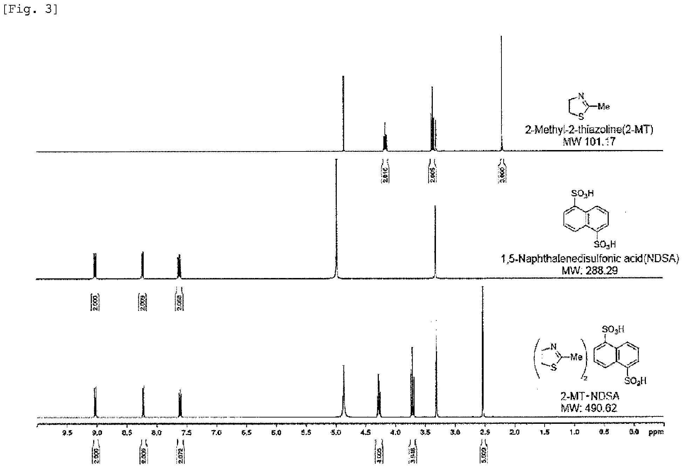

   Fig. 3 shows ¹H NMR spectra (CD₃OD) comparing the 2-MT·hemi-NDSA salt produced in Example 1 with the starting materials.
[Fig. 4]

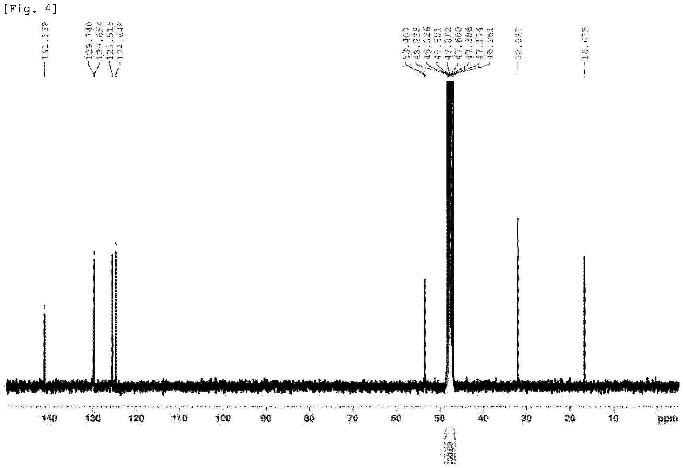

   Fig. 4 is a ¹³C NMR spectrum (CD₃OD) of the hemi-NDSA salt of 2-MT produced in Example 1.
[Fig. 5]

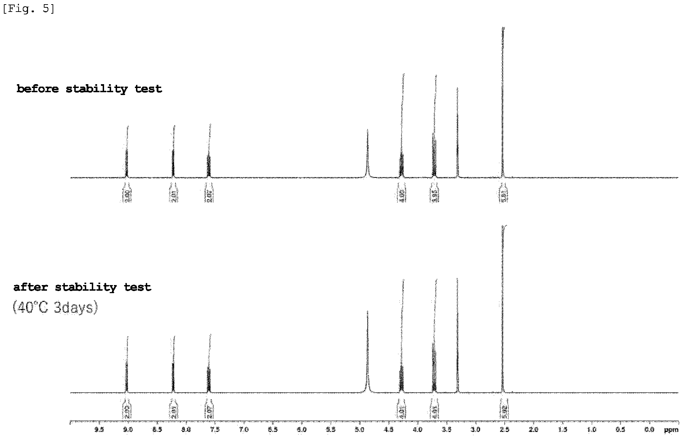

   Fig. 5 shows ¹H NMR spectra (CD₃OD) before and after stability test of the hemi-NDSA salt of 2-MT produced in Example 1.
[Fig. 6]

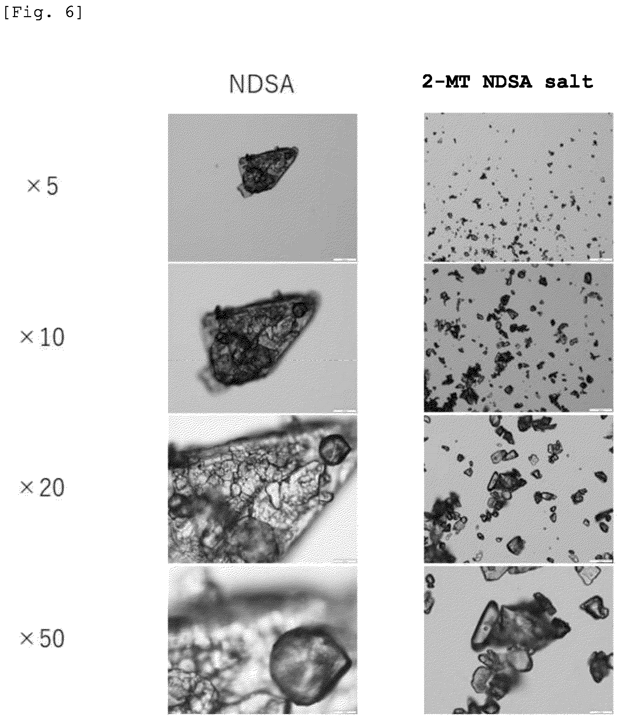

   Fig. 6 shows micrographs of NDSA and 2-MT·hemi-NDSA salt (the number on the left is magnification).
[Fig. 7]

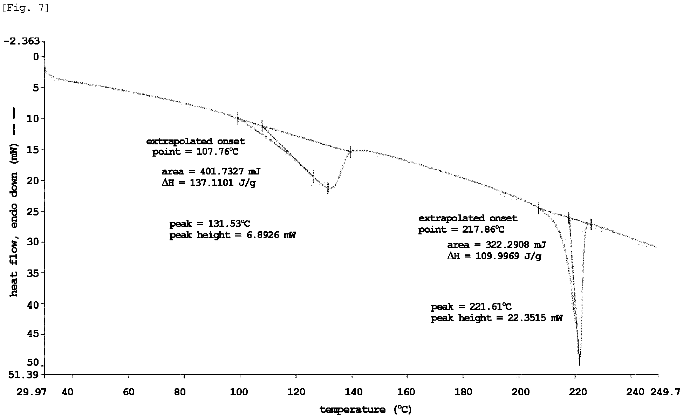

   Fig. 7 shows a differential scanning calorimetry (DSC) chart of 2-MT·hemi-NDSA salt.
[Fig. 8]

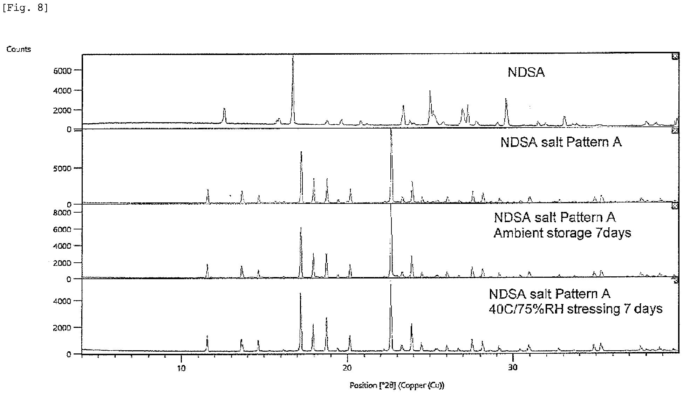

   Fig. 8 shows XRPD patterns of NDSA (the first), 2-MT·hemi-NDSA salt (the second), 2-MT·hemi-NDSA salt post storage at ambient temperature for 7 days (the third), 2-MT·hemi-NDSA salt post storage at 40°C/75% relative humidity for 7 days (the fourth).
[Fig. 9]

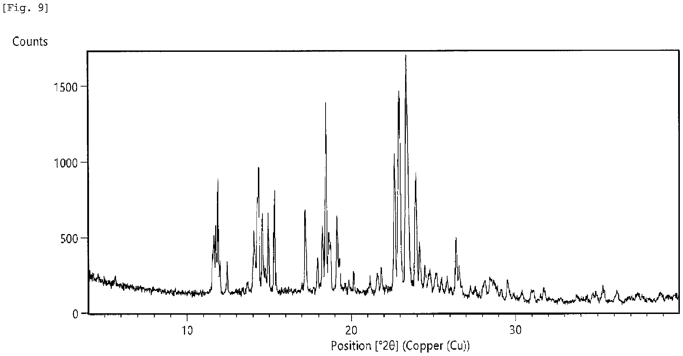

   Fig. 9 shows an XRPD pattern of the mono-NDSA salt of 2-MT produced in Example 2.
[Fig. 10]

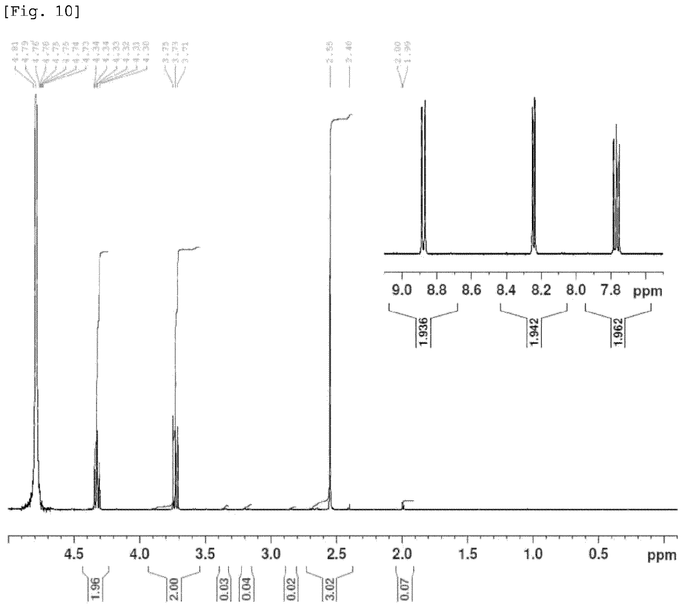

   Fig. 10 shows a ¹H NMR spectrum (D₂O) of the mono-NDSA salt of 2-MT produced in Example 2.
[Fig. 11]

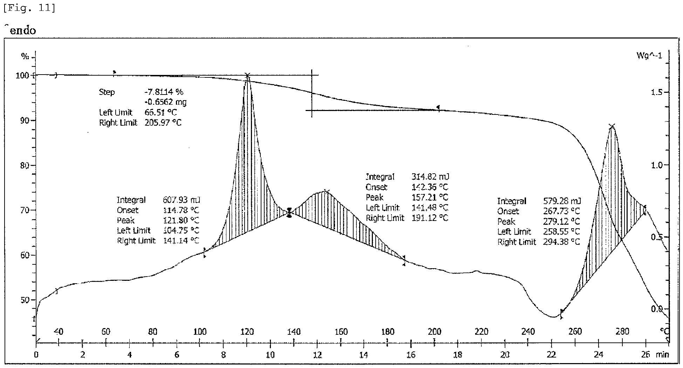

   Fig. 11 shows a thermogravimetric/differential thermal analysis (TG/DTA) thermogram (30-300°C, 10°C/min) of the mono-NDSA salt of 2-MT produced in Example 2.
[Fig. 12]

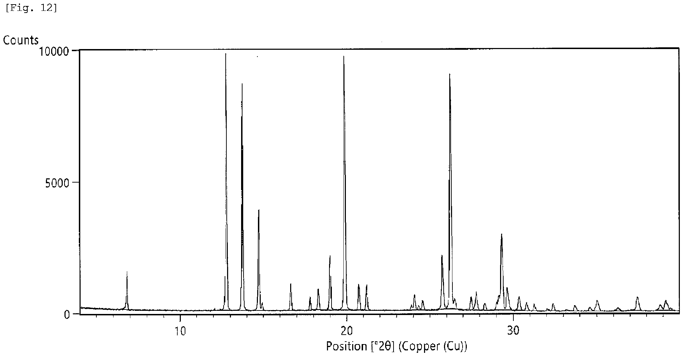

   Fig. 12 shows an XRPD pattern of the monosaccharin salt of 2-MT produced in Example 3.
[Fig. 13]

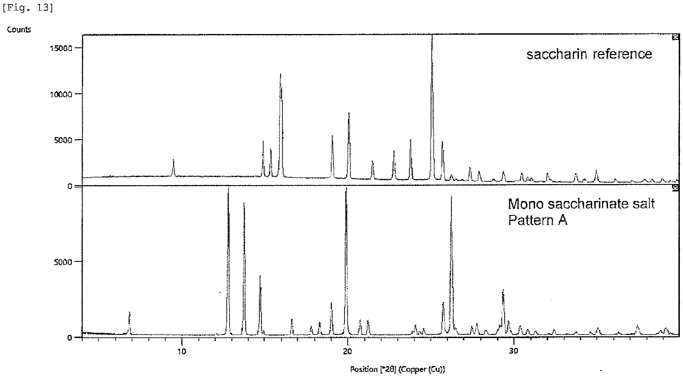

   Fig. 13 shows XRPD patterns comparing the monosaccharin salt of 2-MT produced in Example 3 with the starting material (saccharin).
[Fig. 14]

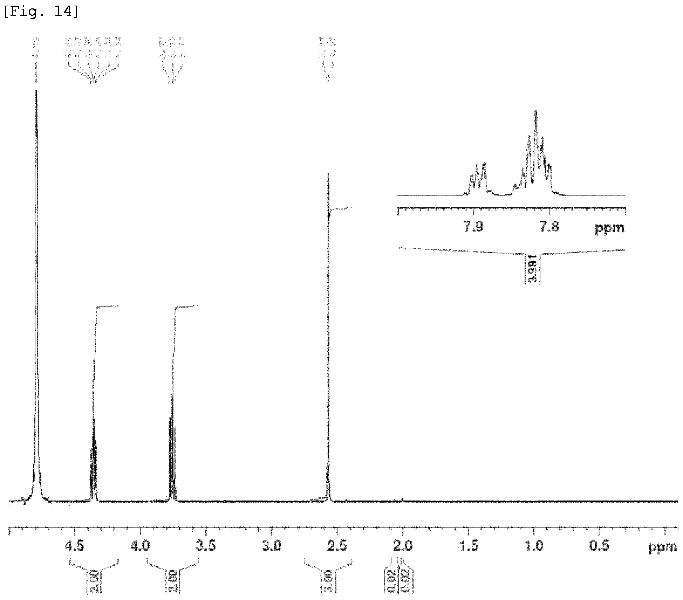

   Fig. 14 shows a ¹H NMR spectrum (D₂O) of the monosaccharin salt of 2-MT produced in Example 3.
[Fig. 15]

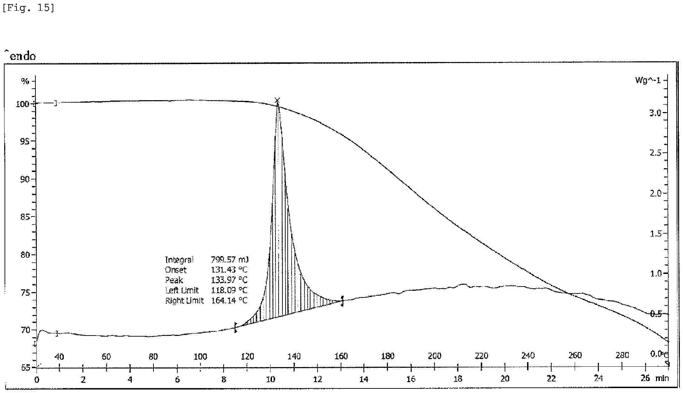

   Fig. 15 shows a TG/DTA thermogram (30-300°C, 10°C/min) of the monosaccharin salt of 2-MT produced in Example 3.
[Fig. 16]

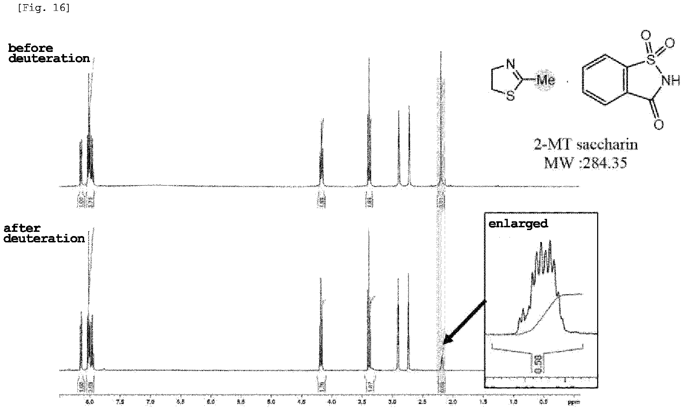

   Fig. 16 shows ¹H NMR spectra (DMF-d₇) of a deuterated form of the monosaccharin salt of 2-MT produced in Example 4 and the monosaccharin salt of 2-MT before deuteration.
[Fig. 17]

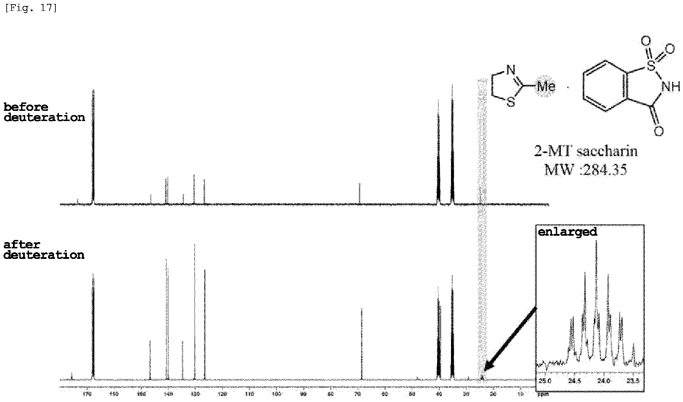

   Fig. 17 shows ¹³C NMR spectra (DMF-d₇) of a deuterated form of the monosaccharin salt of 2-MT produced in Example 4 and the monosaccharin salt of 2-MT before deuteration.
[Fig. 18]

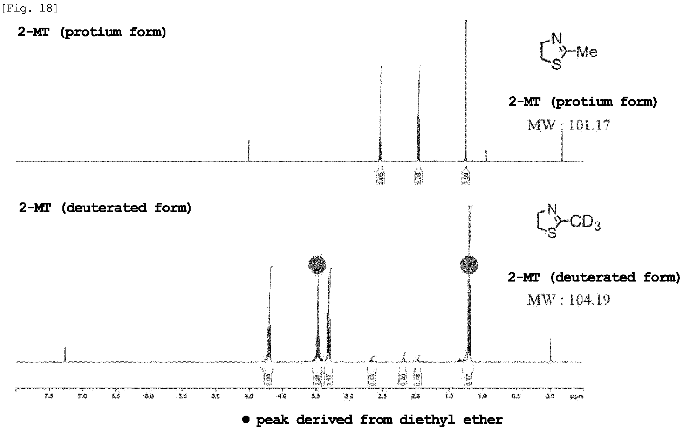

   Fig. 18 shows ¹H NMR spectra (CDCl₃) of a deuterated form (2-methyl-d3-2-thiazoline) of 2-MT produced in Example 5 (1) and a protium form of 2-MT.
[Fig. 19]

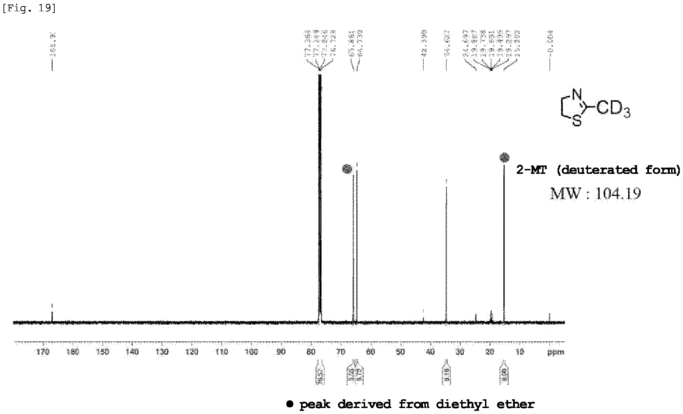

   Fig. 19 shows ¹³C NMR spectrum (DMF-d₇) of a deuterated form (2-methyl-d3-2-thiazoline) of 2-MT produced in Example 5 (1).
[Fig. 20]

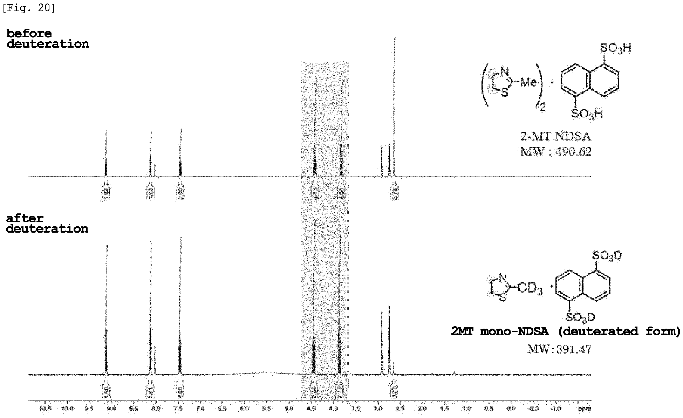

   Fig. 20 shows ¹H NMR spectra (DMF-d₇) of a deuterated form of the mono-NDSA salt of 2-MT produced in Example 5 (2) and the hemi-NDSA salt of 2-MT.
[Fig. 21]

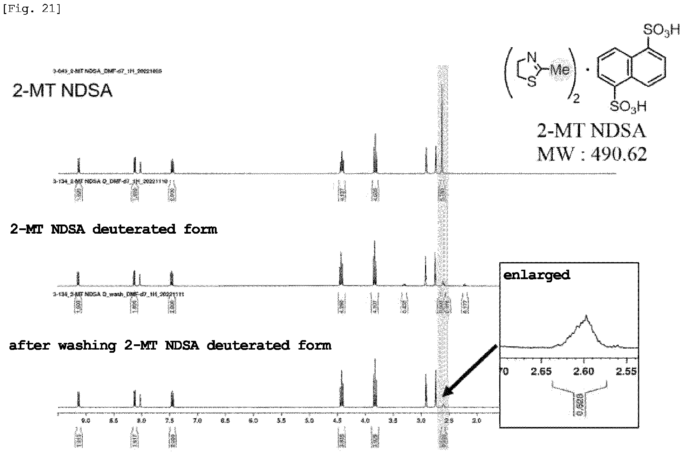

   Fig. 21 shows ¹H NMR spectra (DMF-d₇) of a deuterated form of the hemi-NDSA salt of 2-MT produced in Example 5 (2) and the hemi-NDSA salt of 2-MT.
[Fig. 22]

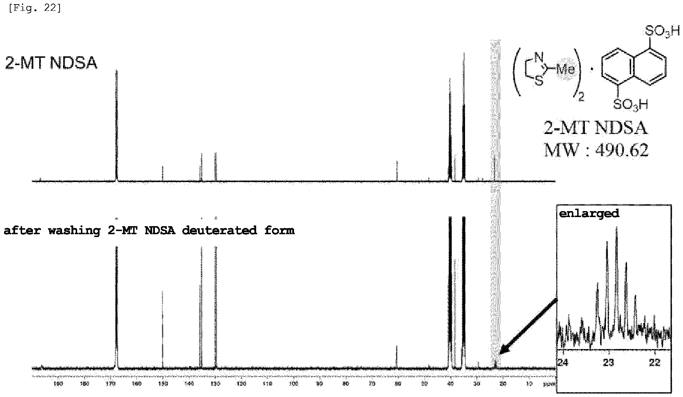

   Fig. 22 shows ¹³C NMR spectra (DMF-d₇) of a deuterated form of the hemi-NDSA salt of 2-MT produced in Example 5 (2) and the hemi-NDSA salt of 2-MT.

### [Description of Embodiments]

The present invention relates to an NDSA salt or a saccharin salt of 2-MT. Saccharin is a compound represented by the formula

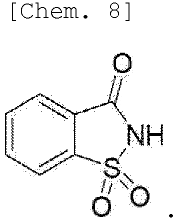

The salt of the present invention may be a solvate, for example, hydrate. Examples of the hydrate include 0.5 hydrate, monohydrate, dihydrate, trihydrate and the like. The salt of the present invention may also be an anhydrate. In the present specification, anhydrate means that the water content is 1.5% by weight or less, preferably 1% by weight or less, as determined by Karl Fischer method, differential scanning calorimetry (DSC), or thermogravimetric/differential thermal analysis (TG/DTA).

In the salt of the present invention, the molar ratio of 2-MT:NDSA or saccharin is, for example, 2:1 to 1:2, 2:1, 1:1, or the like.

The salt of the present invention may be a crystal.

In one embodiment of the present invention, an NDSA salt of 2-MT is provided.

In one embodiment of the present invention, the salt of the present invention is preferably a hemi-NDSA salt of 2-MT or a mono-NDSA salt of 2-MT. The hemi-NDSA salt means a 1/2 NDSA salt in which the molar ratio of 2-MT:NDSA is about 2:1. The mono-NDSA salt is a salt in which the molar ratio of 2-MT:NDSA is about 1:1.

In one embodiment of the present invention, the salt of the present invention is preferably a hydrate of a hemi-NDSA salt of 2-MT or a hydrate of a mono-NDSA salt of 2-MT. Examples of the hydrate include 0.5 hydrate, monohydrate, dihydrate, trihydrate and the like. Preferred examples of the salt of the present invention include a hemi-NDSA salt monohydrate of 2-MT, a mono-NDSA salt dihydrate of 2-MT and the like.

The NDSA salt of 2-MT (including hemi-NDSA salt of 2-MT, hydrate of hemi-NDSA salt of 2-MT, mono-NDSA salt of 2-MT, and hydrate of mono-NDSA salt of 2-MT) may be a crystal.

In one embodiment of the present invention, the hemi-NDSA salt of 2-MT preferably has a powder X-ray diffraction (XRPD) pattern with peaks at 2θ values of 17.2±0.2°, 17.9±0.2°, 18.7±0.2°, and 22.6±0.2°.

In one embodiment of the present invention, the hemi-NDSA salt of 2-MT more preferably has an XRPD pattern with peaks at 2θ values of 11.6±0.2°, 17.2±0.2°, 17.9±0.2°, 18.7±0.2°, 20.1±0.2°, 22.6±0.2°, and 23.9±0.2°.

In one embodiment of the present invention, the hemi-NDSA salt of 2-MT further preferably has an XRPD pattern with peaks at 2θ values of 11.6±0.2°, 13.6±0.2°, 14.7±0.2°, 17.2±0.2°, 17.9±0.2°, 18.7±0.2°, 20.1±0.2°, 22.6±0.2°, 22.7±0.2°, 23.9±0.2°, 27.5±0.2°, and 28.2±0.2°.

In one embodiment of the present invention, the hemi-NDSA salt of 2-MT further more preferably has an XRPD pattern substantially shown in Fig. 1.

In one embodiment of the present invention, the hemi-NDSA salt of 2-MT is characterized in that it preferably has an endothermic peak with an extrapolated onset temperature (onset temperature) of 218°C±3°C, more preferably 218°C±1°C, in differential scanning calorimetry (DSC). The peak temperature of the endothermic peak is preferably 222°C±3°C, more preferably 222°C±1°C.

In one embodiment of the present invention, the hemi-NDSA salt of 2-MT is characterized in that it preferably further has an endothermic peak with an extrapolated onset temperature (onset temperature) of 108°C±3°C, more preferably 108°C±1°C, in differential scanning calorimetry (DSC). The peak temperature of the endothermic peak is preferably 132°C±3°C, more preferably 132°C±1°C.

In one embodiment of the present invention, the hemi-NDSA salt of 2-MT is further more preferably characterized by a DSC chart substantially shown in Fig. 7.

In one embodiment of the present invention, the mono-NDSA salt of 2-MT preferably has a powder X-ray diffraction (XRPD) pattern with peaks at 2θ values of 18.5±0.2°, 22.6±0.2°, 22.9±0.2°, and 23.4±0.2°.

In one embodiment of the present invention, the mono-NDSA salt of 2-MT more preferably has an XRPD pattern with peaks at 2θ values of 11.9±0.2°, 14.4±0.2°, 18.5±0.2°, 22.6±0.2°, 22.9±0.2°, and 23.4±0.2°.

In one embodiment of the present invention, the mono-NDSA salt of 2-MT further preferably has an XRPD pattern with peaks at 2θ values of 11.9±0.2°, 14.4±0.2°, 14.9±0.2°, 15.3±0.2°, 17.2±0.2°, 18.5±0.2°, 19.1±0.2°, 22.6±0.2°, 22.9±0.2°, 23.4±0.2°, and 24.0±0.2°.

In one embodiment of the present invention, the mono-NDSA salt of 2-MT further more preferably has an XRPD pattern substantially shown in Fig. 9.

In one embodiment of the present invention, the mono-NDSA salt of 2-MT is characterized in that it preferably has an endothermic peak with an extrapolated onset temperature (onset temperature) of 268°C±3°C, more preferably 268°C±1°C, in thermogravimetric/differential thermal analysis (TG/DTA). The peak temperature of the endothermic peak is preferably 279°C±3°C, more preferably 279°C±1°C.

In one embodiment of the present invention, the mono-NDSA salt of 2-MT is characterized in that it preferably further has endothermic peaks with extrapolated onset temperatures (onset temperatures) of 115°C±3°C and 142°C±3°C, more preferably 115°C±1°C and 142°C±1°C, in thermogravimetric/differential thermal analysis (TG/DTA). The peak temperatures of the endothermic peaks are preferably 122°C±3°C and 157°C±3°C, more preferably 122°C±1°C and 157°C±1°C.

In one embodiment of the present invention, the mono-NDSA salt of 2-MT is further more preferably characterized by the TG/DTA thermogram substantially shown in Fig. 11.

The purity of the 2-MT NDSA salt (including hemi-NDSA salt and mono-NDSA salt) is preferably 95% or more, more preferably 97% or more, further preferably 98% or more, particularly preferably 99% or more.

In the present specification, unless otherwise specified, the purity refers to purity measured by quantitative analysis using ¹H NMR (proton nuclear magnetic resonance).

In one embodiment of the present invention, a saccharin salt of 2-MT is provided.

In one embodiment of the present invention, the salt of the present invention is preferably a monosaccharin salt of 2-MT. The monosaccharin salt refers to a salt in which the molar ratio of 2-MT:saccharin is about 1:1.

In one embodiment of the present invention, the salt of the present invention is preferably an anhydrate of a monosaccharin salt of 2-MT.

The saccharin salt of 2-MT (including monosaccharin salt of 2-MT, and anhydrate of monosaccharin salt of 2-MT) may be a crystal.

In one embodiment of the present invention, the monosaccharin salt of 2-MT preferably has a powder X-ray diffraction (XRPD) pattern with peaks at 2θ values of 12.8±0.2°, 13.8±0.2°, 19.9±0.2°, and 26.2±0.2°.

In one embodiment of the present invention, the monosaccharin salt of 2-MT more preferably has an XRPD pattern with peaks at 2θ values of 12.8±0.2°, 13.8±0.2°, 14.7±0.2°, 19.9±0.2°, 26.2±0.2°, and 29.3±0.2°.

In one embodiment of the present invention, the monosaccharin salt of 2-MT further preferably has an XRPD pattern with peaks at 2θ values of 6.8±0.2°, 12.8±0.2°, 13.8±0.2°, 14.7±0.2°, 16.6±0.2°, 19.0±0.2°, 19.9±0.2°, 20.7±0.2°, 25.7±0.2°, 26.2±0.2°, and 29.3±0.2°.

In one embodiment of the present invention, the monosaccharin salt of 2-MT further more preferably has an XRPD pattern substantially shown in Fig. 12.

In one embodiment of the present invention, the monosaccharin salt of 2-MT is characterized in that it preferably has an endothermic peak with extrapolated onset temperature (onset temperature) of 131°C±3°C, more preferably 131°C±1°C, in thermogravimetric/differential thermal analysis (TG/DTA). The peak temperature of the endothermic peak is preferably 134°C±3°C, more preferably 134°C±1°C.

In one embodiment of the present invention, the monosaccharin salt of 2-MT is further more preferably characterized by the TG/DTA thermogram substantially shown in Fig. 15.

The purity of the saccharin salt of 2-MT (including monosaccharin salt) is preferably 95% or more, more preferably 97% or more, further preferably 98% or more, particularly preferably 99% or more.

### Deuterated form

The present invention also provides a deuterated form of a NDSA salt or saccharin salt of 2-MT. The term "deuterated form" refers to a compound in which at least one hydrogen (H) in the molecule has been substituted with a deuterium (denoted with ²H or D). The deuteration rate of the deuterated form is preferably 90% or more, more preferably 95% or more, further preferably 98% or more. The deuteration rate can be calculated by the following method. The CHD₂ abundance ratio is calculated from the integral value of the peak corresponding to the methyl group of 2-MT before and after deuteration in the ¹H NMR spectrum, and the deuteration rate can be calculated from the CHD₂ abundance ratio.

As a deuterated form of an NDSA salt or saccharin salt of 2-MT, a deuterated NDSA salt or deuterated saccharin salt of 2-methyl-d3-2-thiazoline in which the methyl group of 2-MT is substituted with a CD₃ group is preferred. Preferred examples of the deuterated form include deuterated forms represented by the following formula (1), (2) and (3), and deuterated hydrates thereof.

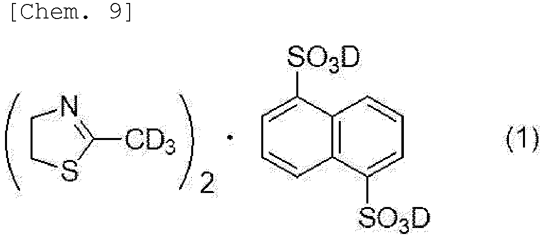

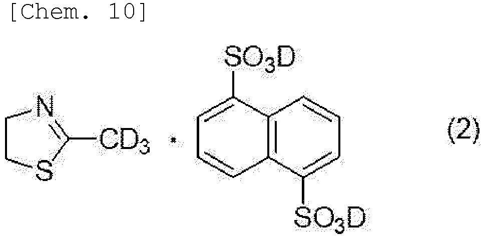

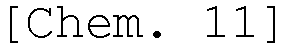

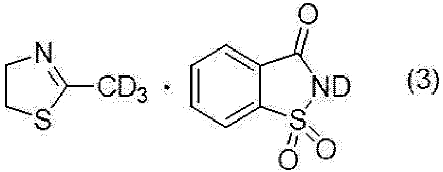

### Production method

The salt of the present invention can be produced, for example, by mixing 2-methyl-2-thiazoline, 1,5-naphthalenedisulfonic acid or saccharin, and a solvent. The obtained solid can be isolated by a known isolation means such as filtration, centrifugation, crystallization, recrystallization and the like.

As the 2-methyl-2-thiazoline, 1,5-naphthalenedisulfonic acid, and saccharin, commercially available ones can be used.

The 1,5-naphthalenedisulfonic acid may be a hydrate, a potassium salt, a sodium salt, a calcium salt or the like. For example, 1,5-naphthalenedisulfonic acid tetrahydrate, 1,5-naphthalenedisulfonic acid disodium dihydrate and the like can be mentioned.

Saccharin may be a hydrate, a potassium salt, a sodium salt, a calcium salt or the like. For example, saccharin potassium, saccharin sodium, saccharin calcium and the like can be mentioned.

Examples of the solvent include, but are not limited to, tetrahydrofuran, acetonitrile, ethanol, isopropyl alcohol, acetone, water, mixtures thereof and the like.

In the embodiments of the present invention, a mixture of an organic solvent such as tetrahydrofuran, acetonitrile, ethanol, isopropyl alcohol, acetone or the like and water can be used as the solvent. The content of water in the solvent is preferably 5 to 30%v/v, more preferably 10 to 20%v/v.

When a 1,5-naphthalenedisulfonic acid salt is produced, preferably tetrahydrofuran, acetonitrile, a mixture of tetrahydrofuran and water, a mixture of acetonitrile and water, and the like, more preferably a mixture of tetrahydrofuran and water, or a mixture of acetonitrile and water, can be used as the solvent. The content of water in the solvent is preferably 5 to 30%v/v, more preferably 10 to 20%v/v.

When a saccharin salt is produced, preferably acetonitrile, tetrahydrofuran and the like, more preferably acetonitrile, can be used as the solvent.

While the volume of the solvent is not particularly limited, the solvent can be used in a volume that renders the concentration of 2-MT in the solvent preferably 0.01 to 3 M, more preferably 0.05 to 2 M, further preferably 0.3 to 1.5 M.

The mixing ratio of 2-MT and 1,5-naphthalenedisulfonic acid or saccharin is preferably 0.1 to 5 mol, more preferably 0.3 to 3 mol, further preferably 0.5 to 2 mol, particularly preferably 0.5 to 1 mol, of 1,5-naphthalenedisulfonic acid or saccharin, per 1 mol of 2-MT.

It is preferable to mix 2-methyl-2-thiazoline, 1,5-naphthalenedisulfonic acid or saccharin, and a solvent, and then allow the mixture to stand or agitate the mixture. The temperature at which the mixture is stood or agitated is not particularly limited, and is preferably -5°C to 35°C, more preferably 0°C to 30°C, further preferably 5°C to 25°C. The time of standing or agitating the mixture is not specifically limited, and is preferably 30 min to 2 days, more preferably 1 to 24 hr.

### Production method (1) of deuterated form

A deuterated form of a saccharin salt of 2-MT can be produced, for example, by mixing a saccharin salt of 2-MT and deuterated methanol, and isolating the obtained deuterated form.

While the volume of deuterated methanol to be used as the solvent is not particularly limited, it can be used in a volume that renders the concentration of an NDSA salt or saccharin salt of 2-MT preferably 30 to 300 mg/mL, more preferably 50 to 200 mg/mL, further preferably 80 to 150 mg/mL.

It is preferable to mix a saccharin salt of 2-MT and deuterated methanol, and then agitate the mixture. The temperature at which the mixture is agitated is not particularly limited, and is preferably 30 to 63°C, more preferably 40°C to 63°C, further preferably 50 to 60°C. The time of agitating the mixture is not specifically limited, and is preferably 30 min to 2 days, more preferably 1 to 24 hr.

The obtained deuterated form can be isolated, for example, by evaporating the solvent from the reaction mixture, washing the obtained solid with a suitable solvent (e.g., acetonitrile), and drying same under reduced pressure. Drying under reduced pressure is preferably performed at room temperature (about 15°C to about 35°C). The obtained deuterated form can also be isolated by known isolation means such as filtration, centrifugation, crystallization, recrystallization and the like.

As the saccharin salt of 2-MT, one produced by the aforementioned production method can be used.

### Production method (2) of deuterated form

A deuterated form of NDSA salt or saccharin salt of 2-MT can be produced, for example, by mixing a deuterated form of 2-MT (2-methyl-d3-thiazoline) and a deuterated form of NDSA or saccharin in a solvent.

As the solvent, preferably tetrahydrofuran, acetonitrile and the like, more preferably tetrahydrofuran, can be used. Alternatively, deuterated methanol can be used as the solvent.

While the volume of the solvent is not particularly limited, the solvent can be used in a volume that renders the concentration of the deuterated form of 2-MT in the solvent preferably 20 to 300 mg/mL, more preferably 30 to 200 mg/mL, further preferably 40 to 150 mg/mL.

The mixing ratio of a deuterated form of 2-MT and a deuterated form of NDSA or saccharin is preferably 0.1 to 5 mol, more preferably 0.3 to 3 mol, further preferably 0.5 to 2 mol, particularly preferably 0.5 to 1 mol, of the deuterated form of NDSA or saccharin, per 1 mol of the deuterated form of 2-MT.

It is preferable to mix a deuterated form of 2-MT, a deuterated form of NDSA or saccharin, and a solvent, and then agitate the mixture. The temperature at which the mixture is agitated is not particularly limited, and is preferably -5°C to 35°C, more preferably 0°C to 30°C, further preferably 5°C to 25°C. The time of agitating the mixture is not specifically limited, and is preferably 30 min to 2 days, more preferably 1 to 24 hr.

The obtained deuterated form can be isolated, for example, by evaporating the solvent from the reaction mixture, washing the obtained solid with a suitable solvent (e.g., tetrahydrofuran), and drying same under reduced pressure. Drying under reduced pressure is preferably performed at room temperature (about 15°C to about 35°C). The obtained deuterated form can also be isolated by known isolation means such as filtration, centrifugation, crystallization, recrystallization and the like.

When a deuterated form of a mono-NDSA salt of 2-MT is obtained, a deuterated form of 2-MT is further added to the obtained deuterated form of a mono-NDSA salt of 2-MT, and the mixture is mixed in a solvent, whereby a deuterated form of a hemi-NDSA salt of 2-MT can be produced. As the solvent, preferably tetrahydrofuran, acetonitrile and the like, more preferably tetrahydrofuran, can be used. Alternatively, deuterated methanol can be used as the solvent.

A deuterated form of 2-MT (2-methyl-d3-thiazoline) can be produced by, for example, reacting a deuterated form of 2-aminoethanethiol hydrochloride (DS-CH₂-CH₂-ND₂·DCl) and deuterated acetonitrile in the presence of sodium deuteroxide according to the method described in Example 5 (1) mentioned later. The deuterated form of 2-aminoethanethiol hydrochloride (DS-CH₂-CH₂-ND₂·DCl) can be produced, for example, by mixing 2-aminoethanethiol hydrochloride and deuterium oxide according to the method described in Example 5 (1) mentioned later.

The deuterated form of NDSA or saccharin can be produced, for example, by mixing NDSA or saccharin, deuterated methanol, and deuterium oxide according to the method described in Example 5 (2) mentioned later.

### Pharmaceutical composition

A 1,5-naphthalenedisulfonic acid salt or a saccharin salt of 2-methyl-2-thiazoline, or a deuterated form thereof (hereinafter also to be referred to as the compound of the present invention) is useful as a medicament, for example, a prophylactic or therapeutic agent for hypoxic injury, ischemia-reperfusion injury or inflammation.

In the present invention, hypoxic injury refers to a disease caused by hypoxia. For example, hypoxia, hypoxic encephalopathy, neonatal hypoxia, hypoxic-ischemic encephalopathy, hypoxic hypoxia, ischemic hypoxia, stagnant hypoxia, altitude sickness, cerebral infarction, myocardial infarction, renal failure, cardiac failure, diabetic angiopathy, arteriosclerosis obliterans, ulcer, spinal cord injury, optic neuropathy, photoreceptor cell injury, neuropathy and the like can be mentioned.

In the present invention, ischemia-reperfusion injury refers to a disorder in cells and tissues of the ischemic organ caused by reperfusion associated with resumption of blood flow to the organ in an ischemic state. For example, ischemia-reperfusion injury after reperfusion therapy for myocardial infarction, cerebral infarction, mesenteric vascular occlusion and the like or after organ transplantation; pressure ulcer and the like can be mentioned.

In the present invention, inflammation refers to a pathological inflammatory condition in the whole body or tissues that is induced by trauma, pathogen invasion, chemical substance stimulation, radiation injury and the like. For example, sepsis, encephalitis, meningitis, arteritis, sinusitis, rhinitis, pneumonia, bronchitis, stomatitis, esophagitis, gastritis, enteritis, hepatitis, myositis, dermatitis, arthritis, nephritis, adrenalitis, lymphangitis, rheumatoid arthritis, psoriasis, osteoporosis, Crohn's disease and the like can be mentioned.

The compound of the present invention can be utilized as a prophylactic or therapeutic agent for hypoxic injury, ischemia-reperfusion injury or inflammation by intracorporeally administering the compound.

### Administration method

The compound of the present invention can be administered to animals, including a human who has or may develop hypoxic injury, ischemia-reperfusion injury or inflammation in an attempt to prevent development of injury or alleviate symptoms. The compound of the present invention can be administered orally at a dose of 1 µg/kg to 5,000 mg/kg. Alternatively, the compound of the present invention can be intracorporeally injected at a dose of 1 µg/kg to 5,000 mg/kg by a method such as intradermal injection, subcutaneous injection, intramuscular injection, intravenous injection, intraarterial injection, intraspinal injection, intraperitoneal injection and the like. Alternatively, the compound of the present invention can be administered at a dose of 1 µg/kg to 5,000 mg/kg, by intranasal administration, pulmonary administration, transdermal administration or rectal administration. The administration frequency may be single-dose administration, or continuous administration at regular intervals, or continuous administration at different time intervals. The animal to be the subject of administration may be, for example, mammals (human, mouse, rat, hamster, rabbit, cat, dog, bovine, sheep, swine, horse, monkey and the like).

When the compound of the present invention is used as a prophylactic or therapeutic agent for hypoxic injury or ischemia-reperfusion injury or inflammation (hereinafter to be also referred to as the agent of the present invention), a pharmaceutically acceptable carrier can be added as necessary. That is, the agent of the present invention can be used as a composition (pharmaceutical composition) containing the compound of the present invention and a pharmaceutically acceptable carrier.

Specific examples of the pharmaceutically acceptable carrier include, but are not limited to, antioxidant, preservative, colorant, flavoring agent, and diluent, emulsifier, suspending agent, solvent, filler, extending agent, buffering agent, delivery vehicle, diluent, carrier, excipient and/or pharmaceutical adjuvant and the like.

The dosage form of the agent of the present invention is not particularly limited and, for example, oral preparation, injection, suppository, ointment, plaster, inhalant, spray and the like can be mentioned. These various administration forms can be each produced by known preparation methods conventionally used by those of ordinary skill in the art.

An injection can be prepared by a method well known in the pertinent field. For example, an injection can be prepared by dissolving in an appropriate solvent (saline, buffer such as PBS, sterile water and the like), sterilizing by filtration with a filter or the like, and then filling in an aseptic container (e.g., ampoule and the like). The injection may contain a conventionally-used pharmacological carrier as necessary. An administration method using a non-invasive catheter can also be adopted. The carrier that can be used in the present invention includes neutral buffered saline, saline containing serum albumin, and the like.

The present invention is explained in more detail in the following by referring to Examples. The Examples do not limit the present invention.

### [Examples]

The abbreviations used in the present specification mean the following.
2-MT: 2-methyl-2-thiazoline
NDSA: 1,5-naphthalenedisulfonic acid
THF: tetrahydrofuran
ACN: acetonitrile

Experiments were carried out at a scale of about 20-30 mg with molar ratios of 1:1 and 2:1 (2-MT: salt former).

### Powder X-ray diffraction (XRPD)

XRPD analyses were performed using a Panalytical Empyrean diffractometer equipped with a Cu X-ray tube and a PIXcel 1D-Medipix3 detector system. The samples were analyzed at ambient temperature in transmission mode and held between low density PVC films. The XRPD program was used (range 3-40° 2θ, step size 0.013°, counting time 99 sec, about 22 min run time/counting time 49 sec, about 11 min run time/counting time 22 sec, about 5 min run time). Samples were spun at 60 rpm during data collection. XRPD patterns were sorted and manipulated using HighScore Plus 2.2c/v4.9 software.

### Differential scanning calorimetry (DSC)

2 to 3 mg of the sample was accurately weighed into an aluminum sample pan and measured in the measurement range of 30 to 250°C at a temperature raising rate of 5°C/min.

### Thermogravimetric/differential thermal analysis (TG/DTA)

Thermogravimetric analyses were carried out on a Mettler Toledo TGA/DSC1 STARe instrument. The calibration standards were indium and tin. Samples were accurately weighed in an aluminum sample pan on an analytical balance and inserted into the TG furnace. The heat flow signal was stabilized for one minute at 30°C, prior to heating to 300°C in a stream of nitrogen at a rate of 10°C/min.

### ¹H nuclear magnetic resonance spectroscopy (NMR)

NMR analysis was carried out on a Bruker 500 MHz instrument.

### Example 1

### Production of hemi-1,5-naphthalenedisulfonic acid salt of 2-methyl-2-thiazoline

2-MT (470 µL, 501.5 mg) was charged into a volumetric flask (5 mL). THF/water (82:18%v/v) was added to concentration 0.99 M. Typical aliquot = 249 µL, about 25 mg.

NDSA tetrahydrate (1 molar equivalent) was charged into a vial. 2-MT (243-257 µL, 1 molar equivalent, THF/water (82:18%v/v)) was added and the mixture was held at ambient temperature (about 20°C) overnight. The solid was isolated by centrifugation and dried with filter paper. The obtained solid was analyzed by powder X-ray diffraction (XRPD), ¹H NMR, and differential scanning calorimetry (DSC). The XRPD pattern is shown in Fig. 1. The main peaks are shown in Table 1.

**[Table 1]**

| diffraction angle [°2θ] | d-spacing [Å] | relative intensity [%] |
|---|---|---|
| 11.6 | 7.66 | 19 |
| 13.6 | 6.49 | 16 |
| 14.7 | 6.04 | 11 |
| 17.2 | 5.16 | 69 |
| 17.9 | 4.94 | 33 |
| 18.7 | 4.74 | 33 |
| 20.1 | 4.41 | 19 |
| 22.6 | 3.93 | 100 |
| 22.7 | 3.93 | 47 |
| 23.9 | 3.72 | 28 |
| 27.5 | 3.24 | 15 |
| 28.2 | 3.17 | 12 |

### Physical properties evaluation

### Confirmation of structure and residual solvent by NMR

The structures were confirmed by ¹H NMR(CD₃OD) and ¹³C NMR(CD₃OD) (Fig. 2 - Fig. 4). The measurement solvent used was deuterated methanol, which dissolves 2-MT·NDSA salt and does not overlap with the solvent peak. ¹H NMR spectrum (CD₃OD) is shown in Fig. 2. The ¹H NMR spectrum (CD₃OD) compared with the starting materials is shown in Fig. 3. The ¹³C NMR spectrum (CD₃OD) is shown in Fig. 4. From the integral values of ¹H NMR, it was confirmed that the molar ratio of the obtained salt was 2 (2-MT:NDSA=2:1). From ¹H NMR, it was confirmed that the residual amount of THF was below the detection limit (about 0.1% or less). From the results of ¹H NMR, impurity was not detected, and it was confirmed that the purity of the obtained salt was 99% or more.

### Solubility

When 2-MT·NDSA salt (20 mg) was added to phosphate buffered saline (PBS pH 7.2 - 7.4, FUJIFILM Wako Pure Chemical Corporation 164-18541, 1 mL) at room temperature, the salt was dissolved easily. The 2-MT·NDSA salt was further added by 80 mg, 100 mg, 100 mg to the solution. By visual observation to confirm dissolution, undissolved remainder was confirmed when the total amount reached 200 mg. Thus, the solubility under the present conditions was 100 mg/mL to 200 mg/mL.

### Stability

2-MT·NDSA salt was placed in a vial, heated to 40°C, and left standing for three days. When this was measured by ¹H NMR, no change in the peaks was observed. Thus, it can be said that 2-MT·NDSA salt is stable in the solid state at 40°C for 3 days (corresponding to 2 weeks at 25°C). The ¹H NMR spectra (CD₃OD) before and after the stability test are shown in Fig. 5.

### Crystallinity

By microscopic observation and DSC measurement, the obtained 2-MT·NDSA salt was considered to be a crystal (Fig. 6 and Fig. 7). The micrographs of NDSA and 2-MT·NDSA salt are shown in Fig. 6 (the number on the left is magnification). The DSC chart of 2-MT·NDSA salt is shown in Fig. 7. Two main peaks were confirmed in the DSC chart shown in Fig. 7. The first endothermic peak had an extrapolated onset temperature (onset temperature) of 107.76°C and a peak temperature of 131.53°C, and the second endothermic peak had an extrapolated onset temperature (onset temperature) of 217.86°C and a peak temperature of 221.61°C. It was considered that the peak on the low temperature side might be water. Therefore, the water content was measured using a moisture meter (Karl Fischer (KF), volumetric method) and found to be 6.78%, confirming that the molar ratio of 2-MT:water was 1:1. Accordingly, the obtained 2-MT·NDSA salt is considered to be a monohydrate of a hemi-1,5-naphthalenedisulfonic acid salt of 2-methyl-2-thiazoline represented by the following formula.

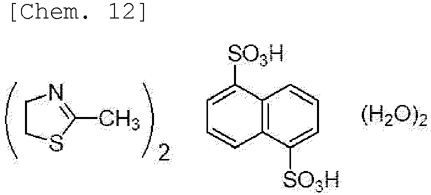

### Deliquescence

A petri dish containing 2-MT·NDSA salt was placed in a relative humidity of about 75%, and left standing in a thermostatic tank at 25°C for 23 hr. No deliquescence was confirmed by visual observation.

### Humidity stress test

A given amount of salt was charged into a vial and placed unsealed in a thermostatic tank at 40°C/75% relative humidity or ambient temperature for 7 days and XRPD analysis was performed. The results are shown in Fig. 8. Fig. 8 shows XRPD patterns of NDSA tetrahydrate (the first), 2-MT·hemi-NDSA salt (the second), 2-MT·hemi-NDSA salt post storage at ambient temperature for 7 days (the third), 2-MT·hemi-NDSA salt post storage at 40°C/75% relative humidity for 7 days (the fourth). As shown in Fig. 8, the 2-MT·hemi-NDSA salt was confirmed to be physically stable post storage at 40°C/75% relative humidity or ambient temperature for 7 days.

### Example 2

### Production of mono-1,5-naphthalenedisulfonic acid salt of 2-methyl-2-thiazoline

NDSA tetrahydrate (0.5 or 1 molar equivalent) was charged into a vial. ACN/water (86:14%v/v, 200-300 µL) was added and mixed for about 5 min. 2-MT (about 23-25 µL, 1 molar equivalent) was added and the mixture was held at ambient temperature (about 20°C) overnight. The solid was isolated by centrifugation and dried with filter paper. The obtained solid was analyzed by powder X-ray diffraction (XRPD), ¹H NMR, and thermogravimetric/differential thermal analysis (TG/DTA).

The sample using 0.5 molar equivalents of NDSA showed an XRPD pattern similar to that of the hemi-NDSA salt of Example 1, and a hemi-NDSA salt was obtained.

The XRPD pattern of the sample using 1 molar equivalent of NDSA is shown in Fig. 9. The main peaks are shown in Table 2. The ¹H NMR spectrum (D₂O) of the same sample is shown in Fig. 10, and the TG/DTA thermogram (30-300°C, 10°C/min) is shown in Fig. 11. XRPD analysis confirmed that the obtained mono-1,5-naphthalenedisulfonic acid salt of 2-methyl-2-thiazoline was a crystal.

**[Table 2]**

| diffraction angle [°2θ] | d-spacing [Å] | relative intensity [%] |
|---|---|---|
| 11.5 | 7.67 | 15 |
| 11.6 | 7.60 | 25 |
| 11.8 | 7.53 | 29 |
| 11.9 | 7.44 | 51 |
| 12.0 | 7.37 | 14 |
| 12.4 | 7.11 | 13 |
| 14.1 | 6.29 | 26 |
| 14.3 | 6.21 | 37 |
| 14.4 | 6.16 | 53 |
| 14.6 | 6.07 | 32 |
| 14.9 | 5.93 | 33 |
| 15.3 | 5.78 | 45 |
| 17.2 | 5.16 | 35 |
| 18.0 | 4.94 | 14 |
| 18.3 | 4.86 | 27 |
| 18.5 | 4.81 | 79 |
| 18.6 | 4.76 | 24 |
| 18.7 | 4.73 | 20 |
| 19.1 | 4.64 | 31 |
| 19.3 | 4.60 | 12 |
| 22.6 | 3.93 | 54 |
| 22.9 | 3.88 | 85 |
| 23.4 | 3.81 | 100 |
| 24.0 | 3.71 | 45 |
| 24.2 | 3.68 | 19 |
| 26.4 | 3.38 | 24 |

From the integral values of ¹H NMR analysis (Fig. 10), it was confirmed that the solid isolated from ACN/water (86:14%v/v) using 1 molar equivalent of NDSA formed a mono-salt, 1:1 (2-MT:NDSA). From the results of ¹H NMR, impurity was not detected, and it was confirmed that the purity of the obtained salt was 99% or more.

TG/DTA analysis (Fig. 11) showed a weight loss of about 7.81% observed between about 66-205°C, which is equivalent to about 2.2 molar equivalents of water, suggesting that the mono-salt is a dihydrate. A related endotherm was observed with an onset temperature of 114.78°C and a peak temperature of 121.80°C, and two endotherms were further observed with an onset temperature of 142.36°C and a peak temperature of 157.21°C, and an onset temperature of 267.73°C and a peak temperature of 279.12°C. Therefore, the obtained 2-MT·NDSA salt is considered to be a dihydrate of a mono-1,5-naphthalenedisulfonic acid salt of 2-methyl-2-thiazoline represented by the following formula.

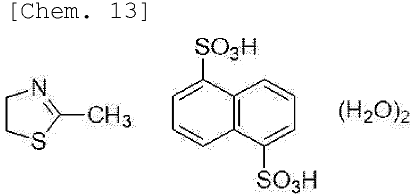

### Example 3

### Production of monosaccharin salt of 2-methyl-2-thiazoline

Saccharin (1 molar equivalent) was charged into a vial. ACN (100-200 µL) was added and mixed for about 5 min. 2-MT (about 23-25 µL, 1 molar equivalent) was added and the mixture was held at ambient temperature (about 20°C) overnight. The solid was isolated by centrifugation and dried with filter paper. The obtained solid was analyzed by powder X-ray diffraction (XRPD), ¹H NMR, and thermogravimetric/differential thermal analysis (TG/DTA). The XRPD pattern is shown in Fig. 12. The main peaks are shown in Table 3. The XRPD pattern compared to the starting material (saccharin) is shown in Fig. 13. The ¹H NMR spectrum (D₂O) is shown in Fig. 14. The TG/DTA thermogram (30-300°C, 10°C/min) is shown in Fig. 15. By XRPD analysis, the obtained monosaccharin salt of 2-methyl-2-thiazoline was confirmed to be a crystal.

**[Table 3]**

| diffraction angle [°2θ] | d-spacing [Å] | relative intensity [%] |
|---|---|---|
| 6.8 | 12.92 | 15 |
| 12.8 | 6.92 | 100 |
| 13.8 | 6.44 | 88 |
| 14.7 | 6.01 | 40 |
| 16.6 | 5.33 | 10 |
| 19.0 | 4.67 | 21 |
| 19.9 | 4.47 | 96 |
| 20.7 | 4.29 | 10 |
| 25.7 | 3.46 | 21 |
| 26.2 | 3.40 | 91 |
| 29.3 | 3.05 | 29 |

From the integral values of ¹H NMR analysis (Fig. 14), it was confirmed that a mono-salt, 1:1 (2-MT:saccharin), was formed. From the results of ¹H NMR, impurity was not detected, and it was confirmed that the purity of the obtained salt was 99% or more.

A weight loss was not observed by TG/DTA analysis (Fig. 15) until after the melt was observed at onset temperature 131.43°C (likely due to the onset of decomposition), suggesting the mono-salt was anhydrous concurring with ¹H NMR analysis. Therefore, the obtained monosaccharin salt is considered to be an anhydrate of a monosaccharin salt of 2-methyl-2-thiazoline represented by the following formula.

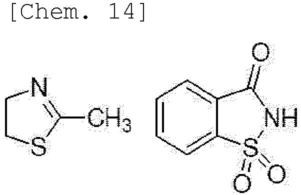

### Example 4

### Production of deuterated form of monosaccharin salt of 2-methyl-2-thiazoline

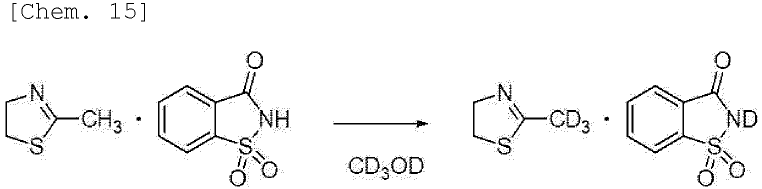

The monosaccharin salt (2.0 g) of 2-MT was dissolved in deuterated methanol (20 mL) and stirred with heating at 60°C. After 18 hr, a portion thereof was sampled, and deuteration was confirmed by NMR, after which the solvent was evaporated. The obtained solid was washed with cooled acetonitrile and dried overnight under reduced pressure at room temperature to obtain a deuterated form (1.02 g) of a monosaccharin salt of 2-MT. The obtained saccharin salt was confirmed by ¹H NRM and ¹³C NMR (Fig. 16 and Fig. 17).

After deuteration, the integral value of the peak (2.21 ppm) corresponding to the methyl group of 2-MT in the ¹H NRM spectrum decreased from 3.01 to 0.58. The deuteration rate of the obtained deuterated form of saccharin salt of 2-MT was 96.73%. In addition, after deuteration, it was confirmed that the peak (24.7 ppm) corresponding to the methyl group of 2-MT coupled at C-D in the ¹³C NMR spectrum.

From the results of ¹H NRM and ¹³C NMR, it was confirmed that a deuterated form of a monosaccharin salt of 2-MT (deuterated monosaccharin salt of 2-methyl-d3-2-thiazoline) (deuteration rate 96.73%) was obtained. From the results of ¹H NMR, impurity was not detected, and it was confirmed that the purity of the obtained salt was 99% or more.

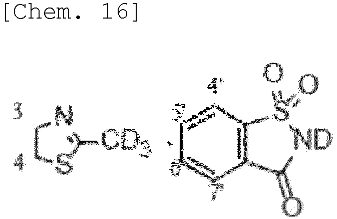

¹H NMR (400 MHz, DMF-d₇) δ ppm 8.15 (dd, J _{7'-6'} = 7.3 Hz, J _{7'-5'} = 1.2 Hz, 1H, 7'), 8.04-7.94 (m, 3H, 4',5',6'), 4.22 (t, J ₃₋₄ = 8.5 Hz, 2H, 3), 3.45 (t, J ₄₋₃ = 8.5 Hz, 2H, 4).

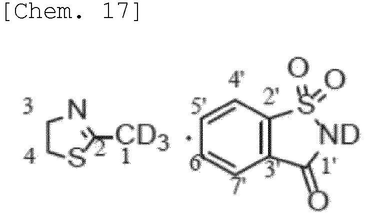

¹³C NMR (100 MHz, DMF-d₇) δ ppm 170.5 (2), 162.8 (1')*, 141.4 (2'), 135.4 (5'), 134.8 (6'), 129.5 (3'), 125.1 (7'), 121.3 (4'), 63.4 (3), 34.4 (4), 19.0 (quintet, J _{1-D} = 20.3 Hz, 1). * The peak of 162.8 (1') may overlap with the carbonyl peak of DMF.

### Example 5

### Production of deuterated form of NDSA salt of 2-methyl-2-thiazoline

### (1) Production of deuterated form of 2-methyl-2-thiazoline (2-methyl-d3-2-thiazoline)

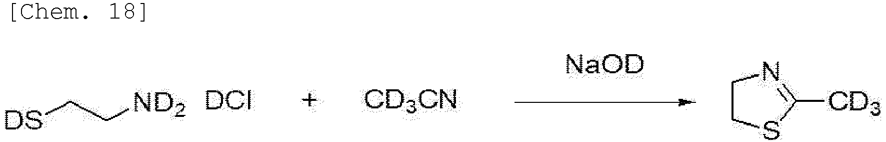

2-Aminoethanethiol hydrochloride (5.68 g) was dissolved in deuterium oxide (5.00 g) and stirred at room temperature for 3 days. The solvent was evaporated and the obtained residue was dissolved in deuterium oxide (5.00 g) and stirred at 50°C for 3 days. The solvent was evaporated, sodium deuteroxide (300 mg) and deuterated acetonitrile (1.74 mL) were added to the obtained residue (deuterated form of 2-aminoethanethiol hydrochloride (DS-CH₂-CH₂-ND₂·DCl)) and the mixture was stirred at 80°C for 3 days. After cooling, the reaction mixture was extracted with diethyl ether (5 mL×10). The organic layer was concentrated at normal pressure (room temperature to 50°C) to obtain a colorless oily deuterated form of 2-methyl-2-thiazoline (2-methyl-d3-2-thiazoline) (2.55 g, yield 74%). From the results of ¹H NRM and ¹³C NMR, it was confirmed that a deuterated form of 2-methyl-2-thiazoline (2-methyl-d3-2-thiazoline) was obtained (Fig. 18 and Fig. 19).

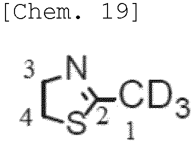

¹H NMR (400 MHz, CDCl₃) δ ppm 4.19 (t, J ₃₋₄ = 8.3 Hz, 2H, 3), 3.30 (t, J ₄₋₃ = 8.3 Hz, 2H, 4).

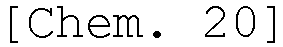

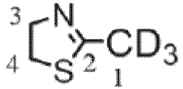

¹³C NMR (100 MHz, DMF-d₇) δ ppm 166.9 (2), 64.7 (3), 34.6 (4), 19.5 (quintet, J _{1-D} = 20.4 Hz, 1).

### (2) Production of deuterated form of NDSA salt of 2-methyl-2-thiazoline

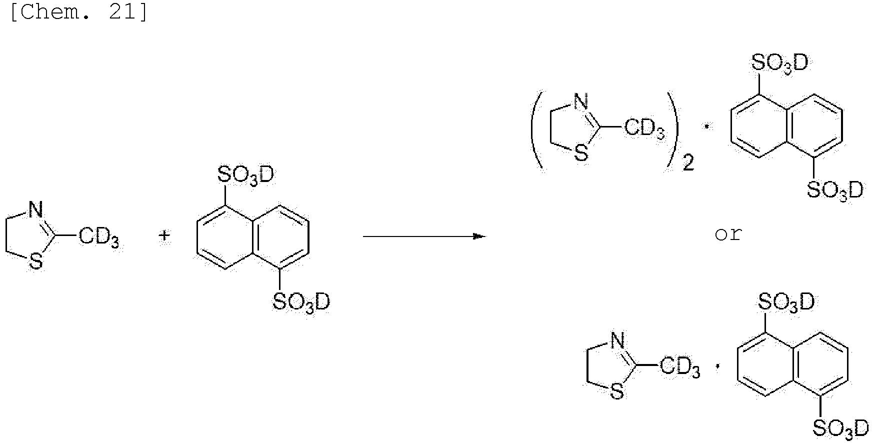

NDSA (4.5 g) was dissolved in deuterated methanol (3.6 mL), deuterium oxide (1.5 mL) was added, and the mixture was stirred. The solvent was evaporated to synthesize a deuterated form of NDSA. The synthesized deuterated form (1.6 g) of NDSA was dissolved in THF (13.3 mL), a deuterated form of 2-MT (2-methyl-d3-2-thiazoline) (0.607 g) was added and the mixture was stirred overnight at room temperature. The solvent was evaporated and the residue was dried under reduced pressure and stirred in THF (5 mL) to recover the solid which was dried under reduced pressure to obtain 1.4 g of a solid. The ¹H NRM of this solid was measured, and the peaks corresponding to CH₂ (3.84, 4.45 ppm) were each missing by 2H. The synthesized NDSA salt (deuterated form) was a mono-NDSA salt of 2-MT (deuterated form) (Fig. 20). Furthermore, a deuterated form of 2-MT was added to synthesize an NDSA salt (deuterated form).

A solution of a mono-NDSA salt of 2-MT (deuterated form) (1.0 g) in deuterated methanol (5.0 mL) was added to the deuterated form of 2-MT (0.61 g). The mixture was stirred for 30 min, and the solvent was evaporated. The residue was dried under reduced pressure at room temperature overnight, and the obtained crystal (1.14 g) was stirred in THF (5 mL) for 5 min under ice-cooling to wash the crystal. The crystal was recovered and dried under reduced pressure overnight at room temperature to obtain 1.09 g of a hemi-NDSA salt of 2-MT (deuterated form). ¹H NRM and ¹³C NMR of the obtained hemi-NDSA salt (deuterated form) of 2-MT were measured (Fig. 21 and Fig. 22).

After deuteration, the integral value of the peak (2.62 ppm) corresponding to the methyl group of 2-MT in the ¹H NRM spectrum decreased from 6 to 0.628. The deuteration rate of the obtained deuterated form of hemi-NDSA salt of 2-MT was 98.27%. In addition, after deuteration, it was confirmed that the peak (23.2 ppm) corresponding to the methyl group of 2-MT coupled at C-D in the ¹³C NMR spectrum.

From the results of ¹H NRM and ¹³C NMR, it was confirmed that a deuterated form of a hemi-NDSA salt of 2-MT (deuterated hemi-NDSA salt of 2-methyl-d3-2-thiazoline) (deuteration rate 98.27%) was obtained.

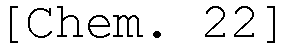

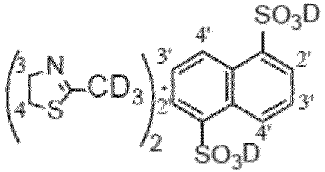

¹H NMR (400 MHz, DMF-d₇) δ ppm 9.12 (dd, J _{2'-3'} = 8.6 Hz, J _{2'-4'} = 0.9 Hz, 2H, 2'), 8.12 (dd, J _{4'-3'} = 7.2 Hz, J _{4'-2'} = 1.1 Hz, 2H, 4'), 7.46 (dd, J _{3'-2'} = 8.6 Hz, J _{3'-4'} = 8.6 Hz, 2H, 3'), 4.44 (t, J ₃₋₄ = 9.3 Hz, 2H, 3), 3.89 (t, J ₄₋₃ = 9.4 Hz, 2H, 4).

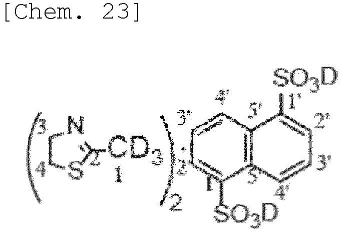

¹³C NMR (100 MHz, DMF-d₇) δ ppm 192.8 (2), 144.4 (1'), 130.4 (5'), 129.8 (2'), 124.7 (4'), 124.2 (3'), 54.4 (3), 32.8 (4), 17.3 (quintet, J _{1-D} = 20.5 Hz, 1).

### [Industrial Applicability]

According to the present invention, a salt of 2-MT, which is a solid excellent in the handling property and stability as an active pharmaceutical ingredient of pharmaceutical products, is provided. The 2-MT salt of the present invention is useful as an active pharmaceutical ingredient of pharmaceutical products. In addition, the deuterated form of a salt of 2-MT is useful as an active pharmaceutical ingredient of pharmaceutical products or a tracer or internal standard substance in biological analyses (e.g., pharmacokinetic analysis and the like).

This application is based on patent application No. 2022-077593 filed in Japan, the contents of which are incorporated by reference in full herein.

## Claims

1. A 1,5-naphthalenedisulfonic acid salt or a saccharin salt of 2-methyl-2-thiazoline, or a deuterated form thereof.

2. The salt according to claim 1, wherein the salt is a 1,5-naphthalenedisulfonic acid salt or a saccharin salt of 2-methyl-2-thiazoline.

3. The salt according to claim 1, wherein the salt is a crystal.

4. The salt according to claim 1, wherein the salt is a 1,5-naphthalenedisulfonic acid salt of 2-methyl-2-thiazoline, or a deuterated form thereof.

5. The salt according to claim 4, wherein the salt is a hemi-1,5-naphthalenedisulfonic acid salt of 2-methyl-2-thiazoline, or a deuterated form thereof.

6. The salt according to claim 5, wherein the salt is a hydrate of a hemi-1,5-naphthalenedisulfonic acid salt of 2-methyl-2-thiazoline.

7. The salt according to claim 5, wherein the salt is a crystal.

8. The salt according to claim 7, wherein the salt has a powder X-ray diffraction pattern with peaks at 2θ values of 17.2±0.2°, 17.9±0.2°, 18.7±0.2°, and 22.6±0.2°.

9. The salt according to claim 7, wherein the salt has a powder X-ray diffraction pattern with peaks at 2θ values of 11.6±0.2°, 17.2±0.2°, 17.9±0.2°, 18.7±0.2°, 20.1±0.2°, 22.6±0.2°, and 23.9±0.2°.

10. The salt according to claim 4, wherein the salt is a mono-1,5-naphthalenedisulfonic acid salt of 2-methyl-2-thiazoline, or a deuterated form thereof.

11. The salt according to claim 10, wherein the salt is a hydrate of a mono-1,5-naphthalenedisulfonic acid salt of 2-methyl-2-thiazoline.

12. The salt according to claim 10, wherein the salt is a crystal.

13. The salt according to claim 12, wherein the salt has a powder X-ray diffraction pattern with peaks at 2θ values of 18.5±0.2°, 22.6±0.2°, 22.9±0.2°, and 23.4±0.2°.

14. The salt according to claim 12, wherein the salt has a powder X-ray diffraction pattern with peaks at 2θ values of 11.9±0.2°, 14.4±0.2°, 18.5±0.2°, 22.6±0.2°, 22.9±0.2°, and 23.4±0.2°.

15. The salt according to claim 1, wherein the salt is a saccharin salt of 2-methyl-2-thiazoline, or a deuterated form thereof.

16. The salt according to claim 15, wherein the salt is a monosaccharin salt of 2-methyl-2-thiazoline, or a deuterated form thereof.

17. The salt according to claim 16, wherein the salt is an anhydrate of a monosaccharin salt of 2-methyl-2-thiazoline.

18. The salt according to claim 16, wherein the salt is a crystal.

19. The salt according to claim 18, wherein the salt has a powder X-ray diffraction pattern with peaks at 2θ values of 12.8±0.2°, 13.8±0.2°, 19.9±0.2°, and 26.2±0.2°.

20. The salt according to claim 18, wherein the salt has a powder X-ray diffraction pattern with peaks at 2θ values of 12.8±0.2°, 13.8±0.2°, 14.7±0.2°, 19.9±0.2°, 26.2±0.2°, and 29.3±0.2°.

21. The deuterated form according to claim 1, which is represented by the formula (1) 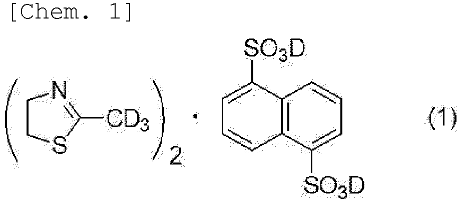 or the formula (2)

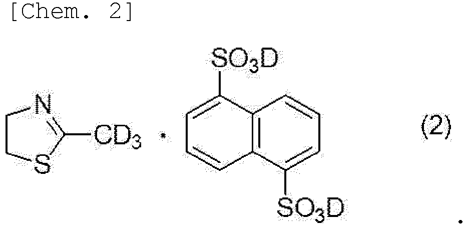

22. The deuterated form according to claim 1, which is represented by the formula (3)

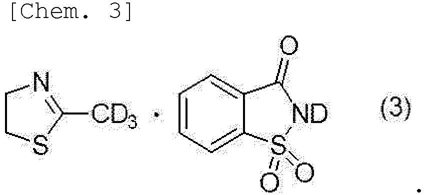

23. A pharmaceutical composition comprising the salt or a deuterated form thereof according to any one of claims 1 to 22, and a pharmaceutically acceptable carrier.

24. A prophylactic or therapeutic agent for hypoxic injury, ischemia-reperfusion injury or inflammation, comprising the salt or a deuterated form thereof according to any one of claims 1 to 22.

25. A method for producing a 1,5-naphthalenedisulfonic acid salt or a saccharin salt of 2-methyl-2-thiazoline, comprising
mixing 2-methyl-2-thiazoline, 1,5-naphthalenedisulfonic acid or saccharin, and a solvent, and
isolating the obtained solid of the 1,5-naphthalenedisulfonic acid salt or saccharin salt of 2-methyl-2-thiazoline.

26. A method for producing a deuterated form of a saccharin salt of 2-methyl-2-thiazoline, comprising
mixing a saccharin salt of 2-methyl-2-thiazoline and deuterated methanol, and
isolating the obtained deuterated form of the saccharin salt of 2-methyl-2-thiazoline.

27. A method for producing a deuterated form represented by the formula (1) 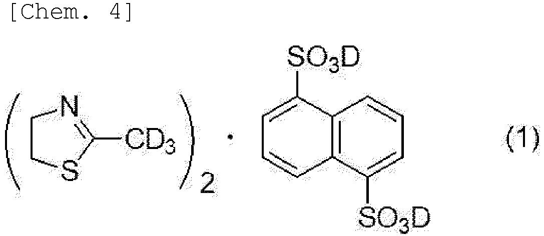 or the formula (2) 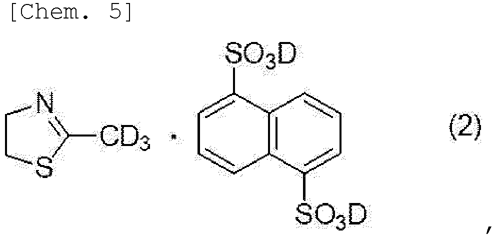 comprising mixing 2-methyl-d3-2-thiazoline and a deuterated form of 1,5-naphthalenedisulfonic acid in a solvent to obtain a deuterated form represented by the formula (1) or the formula (2).

28. A method for producing a deuterated form represented by the formula (3) 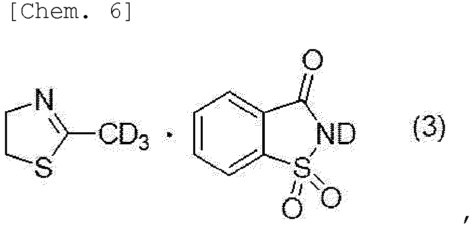 comprising mixing a saccharin salt of 2-methyl-2-thiazoline and deuterated methanol to obtain a deuterated form represented by the formula (3).
